# EUROPEAN PATENT SPECIFICATION

(11) **EP 2 892 342 B1**
(45) Date of publication and mention of the grant of the patent: **12.07.2017**
(21) Application number: 13834854.5
(22) Date of filing: 06.09.2013
(51) Int. Cl.: A01N 43/06, A61F 2/00, A01N 43/16, A01N 43/90, A01N 59/12, A01N 63/02, A61K 33/18, A61K 31/496, A61K 31/7036, A61K 9/19

(54) **AN ANTIBIOFILM AND METHODS FOR MAKING AND USING THE SAME**
ANTIBIOFILM SOWIE VERFAHREN ZU SEINER HERSTELLUNG UND VERWENDUNG
ANTIBIOFILM ET PROCÉDÉS DE FABRICATION ET D'UTILISATION ASSOCIÉS

(30) Priority: 07.09.2012 US 201261697947 P; 05.09.2013 US 201314019152
(43) Date of publication of application: 15.07.2015
(73) Proprietor: GAAB, LLC, University Park, TX 75205 (US)
(72) Inventor: ADAMS, William, P., Dallas, TX 75220 (US); DIGENIS, Alexander, G., Louisville, KY 40241 (US); DIGENIS, George, A., Louisville, KY 40241 (US); DEVA, Anand, Bellevue Hill New South Wales 2023 (AU)
(74) Representative: Zacco Sweden AB
(86) International application number: PCT/US2013/058528
(87) International publication number: WO 2014/039832

(56) References cited:
- WO-A1-2009/012986
- US-A- 6 025 446
- US-A1- 2008 181 950
- DATABASE WPI Week 201306 Thomson Scientific, London, GB; AN 2012-L88068 XP002756293, & IN MUM 201101897 A1 (HELIOS PHARM PVT LTD) 31 August 2012 (2012-08-31)
- FERNANDA GOMES ET AL: "Virulence Gene Expression by Staphylococcus epidermidis Biofilm Cells Exposed to Antibiotics", MICROBIAL DRUG RESISTANCE., vol. 17, no. 2, 1 June 2011 (2011-06-01), pages 191-196, XP055263973, US ISSN: 1076-6294, DOI: 10.1089/mdr.2010.0149
- DATABASE MEDLINE [Online] US NATIONAL LIBRARY OF MEDICINE (NLM), BETHESDA, MD, US; 1994, GAGNON R F ET AL: "Time-kill efficacy of antibiotics in combination with rifampin against Staphylococcus epidermidis biofilms.", XP002756297, Database accession no. NLM7999824 & GAGNON R F ET AL: "Time-kill efficacy of antibiotics in combination with rifampin against Staphylococcus epidermidis biofilms.", ADVANCES IN PERITONEAL DIALYSIS. CONFERENCE ON PERITONEAL DIALYSIS 1994, vol. 10, 1994, pages 189-192, ISSN: 1197-8554
- ELLIOT: 'Role of antimicrobial central venous catheters for the prevention of associated infections' J. ANTIMICROB. CHEMOTHER. vol. 43, no. 4, 1999, pages 441 - 446, XP001042178

## Description

### FIELD OF INVENTION

The present invention relates in general to the prevention of surgical site infection, and in particular, to an anti-infective compound including a polyvinylpyrrolidone (PVP) backbone anchoring two antimicrobials.

### BACKGROUND OF INVENTION

The contamination of surgical sites leading to surgical site infection (SSI) and the fouling of medical prosthetics by bacterial biofilm, device associated infection (DAI), have become recognized as a leading cause of morbidity and mortality worldwide. SSI's also represent a substantial economic burden. In the United Kingdom, up to eight percent of hospitalized patients develop SSI, as reported in Leaper DJ. Risk factors for and epidemiology of surgical site infections. Surgical Infections 2010; 11(3): 283-7. The CDC reported a rate of two SSIs per 100 procedures in US hospitals during 2002 with attributable costs ranging from $3000-$5000 (U.S.) per procedure in multi-specialty studies. See, Klevens RM, Edwards JR, Richards CLJ. Estimating health care-associated infections and deaths in US hospitals, 2002. Public Health Report 2007;122(2): 160-6; and Hollenbeak CS, Murphy D, Dunagan WC. Nonrandom selection and the attributable cost of surgical site infections. Infection Control Hospital Epidemiology 2002; 234(2): 177-82.

Another long-term study by the Inter-regional Co-ordination Centre for Nosocomial Infection Control (INCISO) Network Study group reported that 38% of the deaths that occurred in patients with an SSI were directly attributable to the infection. See, Astagneau P, Rioux C, F. G. Morbidity and mortality associated with surgical site infections. Journal of Hospital Infection 2001; 28(4): 267-74. Patients are also more likely to have extended hospital admissions, increased expenditure and higher rates of readmissions, as reported in Jenney AWJ, Harrington GA, Russo PL. Cost of surgical site infections following coronary artery bypass surgery. ANZ Journal of Surgery 2001; 71(11): 662-4. In addition, loss of productivity of the patients adds to the economic burden. A systematic review of the epidemiological and economic burden of SSI in Europe estimated that SSIs cost European health care systems between €1.47 billion and €19.1 billion each year. *Leaper,* Id.

DAI's have now been documented in a range of medical prosthetics including:
- K wires, orthopedic fixation, as reported in Dobbins JJ, Seligson D, Raff MJ. Bacterial colonisation of orthopaedic devices in the absence of clinical infection. Journal of Infectious Diseases 1988; 158: 203-5 and Ehrlich GD, Stoodley P, Kathju S, et al. Engineering approaches for the detection and control of orthopaedic biofilm infections. Clinical orthopaedics and related research 2005; (437): 59-66;
- joint prostheses, as reported in Ramage G, Tunney MM, Patrick S, Gorman SP, Nixon JR. Formation of propionibacterium acnes biofilms on orthopaedic biomaterials and their susceptibility to antimicrobials. Biomaterials 2003; 24(19): 3221-7 and Tunney MM, Dunne N, Einarsson G, McDowell A, Kerr A, Patrick S. Biofilm formation by bacteria isolated from retrieved failed prosthetic hip implants in an in vitro model of hip arthroplasty antibiotic prophylaxis. J Orthop Res 2007; 25(1): 2-10;
- contact lenses, as reported in Donlan RM, Costerton JW. Biofilms: survival mechanisms of clinically relevant microorganisms. Clinical microbiology reviews 2002; 15(2): 167-93;
- indwelling urinary catheters, as reported in Donlan RM. Biofilms and device-associated infections. Emerging Infectious Diseases 2001; 7(2): 277-81; and
- breast implants, as reported in Tamboto H, Vickery K, Deva AK. Subclinical(biofilm) infection causes capsular contracture in a porcine model following augmentation mammaplasty. Plastic and Reconstructive Surgery 2010; 126: 835-42 and Pajkos A, Deva AK, Vickery K, Cope C, Chang L, Cossart YE. Detection of subclinical infection in significant breast implant capsules. Plastic and Reconstructive Surgery 2003; 111 (5): 1605-11.

Bacteria derived from skin, such as coagulase negative *Staphylococcus epidermidis*, derive their pathogenesis primarily from their ability to form biofilms on the surfaces of indwelling medical devices, as reported in: Tunney, Id.; Donlan, Id.; O'Gara JP, Humphreys H. Staphylococcus epidermidis biofilms: importance and implications. Journal of medical microbiology 2001; 50(7): 582-7; Costerton JW, Montanaro L, Arciola CR. Biofilm in implant infections: its production and regulation. The International Journal of Artificial Organs 2005; 28(11): 1062-8; and Vinh DC, Embil JM. Device-related infections: a review. Journal of Long-term Effects of Medical Implants 2005; 15(5): 467-88.

Formation of biofilms occurs in two phases. Rapid primary attachment of bacterial cells to the polymer surface is followed by a more prolonged accumulation into multilayered cell clusters in a growth-dependent process. As adherent bacteria divide and produce extracellular polymeric substance locally, they form a highly structured matrix-enclosed microcolony. The glycocalyx matrix serves to hold the microcolony together and to anchor it to the substratum or to other microcolonies. This process is particularly relevant because biofilm associated organisms are much more resistant to antimicrobial agents than are planktonic (free swimming or non biofilm) organisms, See, Donlan RM. Role of biofilms in antimicrobial resistance. Asaio J 2000; 46(6): S47-52; Donlan RM. Biofilm formation: a clinically relevant microbiological process. Clin Infect Dis 2001; 33(8): 1387-92; and Gilbert P, McBain AJ. Biofilms: their impact on health and their recalcitrance toward biocides. American Journal of Infection Control 2001; 29(4): 252-5. Once formed, associated inflammation around the contaminated prosthesis will result in a high risk of medium to long-term device failure. It has been estimated that the cost of revisional surgery in failed orthopedic devices alone is estimated at $1 billion (U.S.) per year, as reported in Von Eiff C, Jansen B, Kohnen W, Becker K. Infections Associated with Medical Devices : Pathogenesis, Management and Prophylaxis. Drugs 2005; 65(2): 179-214; and Costerton JW. Biofilm theory can guide the treatment of device-related orthopaedic infections. Clinical Orthopaedics and Related Research 2005; (437): 7-11. Strategies to reduce SSI and DAI by developing an effective antibiofilm agent for use in surgery are currently being investigated. See: Klemm P, Hancock V, Kvist M, Schembri MA. Candidate targets for antivirulence drugs : selected cases of bacterial adhesion and biofilm formation. Future Microbiology 2007; 2(6): 643-53; and Kiedrowski MR, Horswill AR. New approaches for treating staphylococcal biofilm infections. Annals of the New York Academy of Sciences 2011; 1241(1): 104-21. To date, whilst a number of agents have been trialed, there are no clinically proven compounds that have been shown to be effective at both prevention and/or treatment of biofilm infection, as reported in Vasiley K, Cook J, Griesser HJ. Antibacterial surfaces for biomedical devices. Expert Review of Medical Devices 2009; 6(5): 553-67.

Thomson Scientific (GB; AN 2012-L88068 XP002756293,& IN MUM 201101897 Al, Povidone-iodine and chitosan containing dressing ointment and process of manufacturing same. (HELIOS PHARM PVT LTD) 31 August 2012) discloses a biodegradable pharmaceutical grade dressing ointment for treating wounds, cuts, abrasions, surgical sutures and any such injuries, in the form of topical hemostatic-antiseptic dressing ointment, which essentially comprises chitosan and povidone-iodine as active substances which altogether synergizes the effect, as the chitosan serves the purpose of topical hemostasis with antimicrobial activity for arresting the bleeding and efficiently promoting the faster healing whereas povidone-iodine enhances the wound healing property by preventing the contamination and infection at the affected site.

As reported in FERNANDA COMES ET AL ("Virulence Gene Expression by Staphylococcus epidermidis Biofilm Cells Exposed to Antibiotics", Microbial Drug resistance, vol. 17, no. 2, 1 June 2011 (2011-06-01), pages 191-196), their results showed that normally the expression of *icaA* is accompanied by the expression of *rsbU.* Further, in addition to the already known advantages of antibiotic combinatorial therapy, their results show that the use of rifampicin combined with gentamicin and clin-damycin may be a better therapy strategy and more effective than rifampicin alone (the most efficient antibiotic against *S*. *epidermidis* used in clinical practice). The combination therapy, in addition to allowing for an almost equally ef- fective killing of bacterial cells, results in lower expression of *icaA* and *rbsU,* and consequently less PIA production, than if rifampicin was used alone, thereby ensuring a more efficient control of *S*. *epidermidis* biofilm-associated infections.

Gagnon R F et al. ("Time-kill efficacy of antibiotics in combination with rifampin against Staphylococcus epidermidis biofilms "Advances in peritoneal dialysis. Conference on peritoneal dialysis" 1994,vol. 10, 1994, pages 189-192), demonstrated that experimental biofilms of Staphylococcus epidermidis, the main pathogen associated with implantation, are exquisitely sensitive to the action of rifampin. These results are relevant to the design of optimal therapeutic regimens for the management of resistant implant-associated infections.

WO 2009/012986 discloses the use of a pharmaceutical composition for the local treatment or prevention of a tissue infection at an infection site, the composition comprising at least two different antibiotics of group A or pharmaceutically acceptable derivatives thereof, or an antibiotic of group A, and at least one antibiotic of group B or pharmaceutically acceptable derivatives thereof. Further discussed is the use of a combination of at least one antibiotic acting as an inhibitor or bacterial RNA polymerase and at least one antibiotic affecting the bacterial cell wall or its synthesis as anti-adhesive against microorganisms on surfaces.

### SUMMARY OF INVENTION

The principles of the present invention are embodied in a novel antibiofilm complex using a polyvinylpyrrolidone (PVP) backbone to anchor two antimicrobials. The antibiofilm advantageously may be used an anti-infective compound in both the treatment and prevention of bacterial contamination of medical prosthetics and in the preparation of skin, mucosa and other surgical sites prior to performing invasive procedures and deployment of medical devices. The significant advantages of the embodiments of the present principles include: (1) solubility; (2) biologic safety at working concentrations; (3) broad spectrum of activity; (4) a synergistic effect of antimicrobial compounds to penetrate and kill biofilm; (5) a synergistic effect to overcome known mechanisms of antimicrobial resistance; (6) and proven efficacy *in vitro* and *in vivo.* The novel antibiofilm complex has been designated GAAB-1.

### BRIEF DESCRIPTION OF DRAWINGS

For a more complete understanding of the present invention, and the advantages thereof, reference is now made to the following descriptions taken in conjunction with the accompanying drawings, in which:
FIGURE 1 is a diagram showing the grappling of iodine (I₃) by a PVP j-lactam hydrophilic ring attached to hydrophobic exocyclic aliphatic chain backbone to form an antibiofilm complex according to a preferred embodiment of the present invention; and
FIGURE 2 is a diagram comparing the activity of the antibiofilm complex with the activity of betadine when directed against certain common biofilm forming pathogens *in vitro.*

### DETAILED DESCRIPTION OF THE INVENTION

The principles of the present invention are embodied in a potent and novel antibiofilm product capable of reducing the rates of subsequent SSI and DAI when used as a prophylactic agent. The product can also be applied as a therapeutic agent to decrease the bacterial biofilm load on established DAI. The product will be delivered as an aqueous solution. Other formulations include slow release foam, coating for surfaces and a Nano-crystalline product.

### Composition and Synthesis

The preferred embodiment of the present invention is a coprecipitate of polyvinylpyrrolidone (PVP) with iodine and the two antibiotics Rifampin and Tobramycin. Rifampin is a semi synthetic derivative of rifamycin B. Rifamycins are a group of macrocyclic ring antibiotics obtained from streptomyces mediterranei. Many of the members of their chemical class, including their semi synthetic derivatives Rifampin and Rifabutine, possess broad spectrum antimicrobial activity and most notably against gram positive bacteria. They are also active against some gram-negative bacteria and many viruses. Rifampicin is relatively insoluble in water. Gentamicin and Tobramycin are broad spectrum antibiotics belonging to a large chemical class called aminoglycosides. They have a broad spectrum of activity against many common pathogens and in particular they possess a high degree of activity against *Pseudomonas aeruginosa* and other gram-negative bacteria. They are freely soluble in water.

In the preferred embodiment, the coprecipitate is a lyophilized powder, which was found to exhibit pronounced synergistic activity against gram positive and negative bacteria. In a preferred embodiment, the coprecipitate was formulated to contain the following ingredients in 50:50 water for injection:

| | |
|---|---|
| TBA (tertiary butyl alcohol) | 5 to 45%, typically 5 to 25% |
| Rifampin | 2.5g |
| Tobramycin | 80mg |
| Povidone-iodine (10%) | 1.2g |

The resulting solution was freeze dried according to the lyophilization cycle shown in the Table 1, which describes the times/temperatures and pressure utilized in the lyophilization process. The latter procedure of freeze-drying is a critical parameter for the production of the product and its resulting antimicrobial activity.

**Table 1: A Preferred Method For Producing A Complex Antibiofilm By Coprecipitation**

| **Step** | **Temp. (°C)** | **Ramp (°C / min)** | **Pressure (mTorr)** | **Time** | |
|---|---|---|---|---|---|
| | | | | **Hours** | **Minutes** |
| Loading | RT | | | | |
| Freezing | -25 | 0.5-1.0 | | 1 | 30 |
| | -25 | Hold | | 10 | 00 |
| | -45 | 0.1-0.5 | | 3 | 00 |
| | -45 | Hold | | 5 | 00 |
| Evacuation | -45 | | 100 | 0 | 30 |
| Primary Drying | 20 | 0.5-1.0 | 100 | 2 | 00 |
| | 20 | Hold | 100 | 30 | 00 |
| Secondary Drying | 40 | 0.5-1.0 | 150 | 1 | 00 |
| | 40 | Hold | 150 | 12 | 00 |
| Stoppering | 25 | 0.5-1.0 | 150 | 1 | 00 |
| Unloading | | | | | |

The antibiofilm complex, which has been designated GAAB-1, may be diluted at least seven times and maintain its synergistic efficacy in treating and reducing the development of biofilms. The most concentrated range of the product components include Rifampin 2.5 gms, Tobramycin or Gentamicin 80mg, and PVP-I 1.2 gms in a solution of 520 ml. This would produce a concentration of 4,800 micrograms/ml of Rifampin, 154 micrograms/ml of Tobramycin or Gentamicin, and 2,300 micrograms of PVP-I (with essentially 23 micrograms or 1% of free iodine). At seven times dilution the concentrations would be 686 micrograms/ml of Rifampin, 22 micrograms/ml of Tobramycin or Gentamicin and 3.2 micrograms/ml of PVP-I. Greater dilutions produces more free iodine but may create instability in the copreciptiate's synergistic qualities.

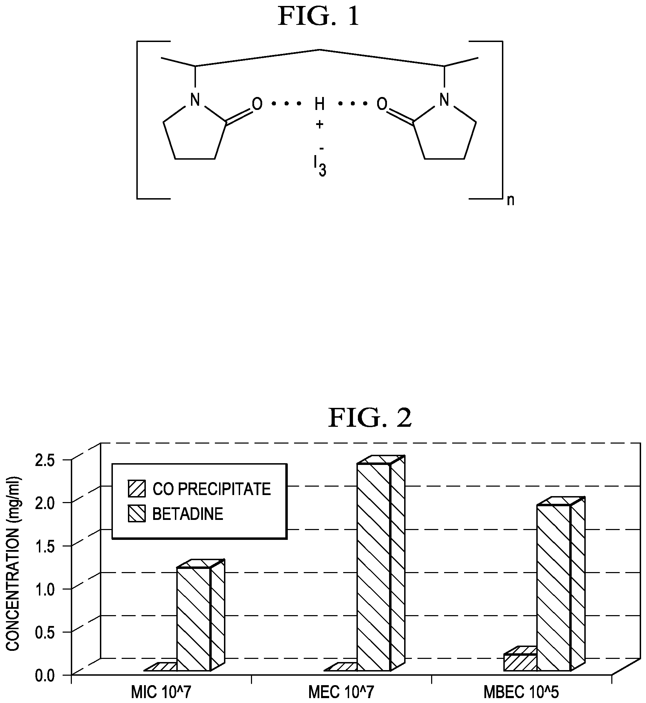

The coprecipitate is a novel antibiofilm complex capable of reducing the rates of subsequent SSI and DAI when used as a prophylactic agent. This antibiofilm complex was tested against common bacterial pathogens both in planktonic and biofilm states in vitro, as discuss further below. Specifically, the novel antibiofilm complex is composed of a polyvinylpyrroloidone (PVP) backbone, which grapples iodine and two antibiotics (Rifampicin and Gentamycin). Figure 1 illustrates the grappling of I₃ by a PVP j-lactam hydrophilic ring attached to hydrophobic exocyclic aliphatic chain backbone.

Advantageously, PVP is an amorphous polymer that readily reacts with drugs, dyes and electron acceptors. PVP additionally increases the presence of a highly polar amide group and apolar methylene and methine groups. (See, de Faria DLA, Gil HAC, de Quieroz AAA. The Interaction Between Polyvinylpyrrolidone and 12 as Probed by Raman Spectroscopy. Journal of Molecular Structure 1999; 479: 93-8. ) It is non-toxic and was employed as a plasma expander in World War II and as a cryopreservant for red blood cells, as reported in Ford JL. The Current Status of Solid Dispersions. Pharm Acta Helv 1986; 3: 69-88.

In other words, according to the present inventive principles, PVP is combined with relatively insoluble antiseptics and antibiotics. The resultant co-precipitate antibiofilm complex acts as a directed chemical" smart bomb" able to deliver its payload of antibiotics and antiseptics deep into the bacterial biofilm and thereby producing higher kill rates for comparatively lower dosages. In addition, the novel antibiofilm complex provides a number of other significant advantages in drug delivery, including: (1) membrane seeking capabilities (e.g., the penetration of biofilm exopolysaccharide); (2) higher water solubility; (3) fixed concentrations of antibiotic/antiseptic components thereby standardizing the dosage of delivery; and (4) stability with slow release of antimicrobial activity providing continued antibiofilm protection after delivery. Furthermore, the present inventive principles can be extended to produce a family of stable chemical "smart bombs" tailored to suit a specific antimicrobial or antiviral target. This will allow for design of an array of anti-infective weaponry to cater for future changes in microbial resistance patterns.

### Experimental Results

The antibiofilm activity of the novel antibiofilm complex was directed against the four most common biofilm forming pathogens *in vitro.* The results of the experimentation are as follows.

*Definitions.* The following definitions apply to the discussion of the experimental results:
*Planktonic bacteria:* bacteria growing free in suspension;
*Biofilm bacteria:* a structured community of bacterial cells enclosed in a self-produced exo-polysaccharide matrix that serves to attach them to a solid surface and to each other;
*MIC:* Minimum Inhibitory Concentration is the concentration of an antimicrobial that is required to inhibit the growth of a standardized inoculum of planktonic cells; *MEC:* Minimum Eradication Concentration is the concentration of an antimicrobial that is required to kill all the organisms of a standardized inoculum of planktonic cells;
*MBIC:* Minimum Biofilm Eradication Concentration is the concentration of an antimicrobial that is required to inhibit the growth of a standardized number of organisms growing as a biofilm; and
*MBEC:* Minimum Biofilm Eradication Concentration is the concentration of an antimicrobial that is required to kill a standardized number of organisms growing as a biofilm.

*Test Organisms.* The antibiofilm activity of the novel antibiofilm complex was directed against the four most common biofilm forming pathogens, namely:
1. *Staphylococcus epidermidis* strain ATCC 35984;
2. *Staphylococcus aureus* strain ATCC 25932 (MSSA);
3. Methicillin resistant *Staphylococcus aureus* strain ATCC 43300 (MRSA); and
4. *Pseudomonas aeruginosa* strain ATCC 25619.

*Independent Test Solution.* The independent test solution consisted of Betadine Batch 801070. Povidone-iodine antiseptic solution containing povidone iodine 10% w/v equivalent to 1%w/v available iodine.

*Test Parameters.* The testing parameters were:
1. Determining the efficacy the co-precipitate (antibiofilm complex) versus common biofilm forming bacteria at the 1-hour time point following reconstitution, as measured by the MIC, MEC, MBIC, MBEC;
2. Co-precipitate (antibiofilm complex) versus S. *aureus* at the 3 hour time point following reconstitution (MIC, MEC, MBIC, MBEC);
3. Physical mixture of PVP, iodine, gentamycin and rifampicin versus *S*. *aureus* at the 1, 3 and 24 hour time points following reconstitution (MIC, MEC, MBIC, MBEC);
4. The efficacy of Betadine (povidone iodine) against *S*. *aureus* and *S*. *epidermidis* immediately following dilution (MIC, MEC, MBIC, MBEC);
5. The efficacy of gentamycin against *S*. *aureus* (MIC, MEC, MBIC, MBEC); and
6. The efficacy of Rifampicin against *S*. *aureus* (MIC, MEC, MBIC, MBEC).

*Experimental Protocol for MIC and MEC.* The protocol for the determination of MIC and MEC was:
Day 1: Inoculate one colony of bacteria into 100% Tryptone Soy Broth (TSB) and 37°C shaking incubation overnight.
Day 2: Dilute bacteria to give 10⁷, 10⁶, 10⁵ bacteria/well and dispense in 96 well plates. Test product is added as per experimental protocol immediately or at 1 hour or 3 hours.
Day 3: Read MIC plates. Transfer 20µl from each well of the MIC plate that shows no growth to 180µl of fresh TSB in the MEC plate.
Day 4: Read MEC plates.

*Experimental Protocols for MBIC and MBEC.* The protocol for the determination of MBIC and MBEC was:
Day 1: Inoculate one-colony bacteria into 100% Tryptone Soy Broth (TSB) and 37°C shaking incubation overnight.
Day 2: dilute culture to give 10⁶ bacteria/ml and dispense into 96 well plates and incubate overnight to give 10⁷ attached cells/well 9 (MBIC plate).
Day 3: Remove planktonic cells from MBIC plate by washing wells and add fresh media and antibiofilm products.
Day 4: Read MBIC plates. Add 180ul fresh TSB in a new 96 well plate (MBEC plate) and transfer 20µl from each well of the MBIC plate that shows no growth to fresh MBEC plate.
Day 5: Read MBEC plates.

### Experiment 1. The efficacy of the co-precipitate (antibiofilm complex) versus common biofilm forming bacteria at the 1-hour time point following reconstitution.

The efficacy of the novel antibiofilm complex was measured against the bacteria *S*. *epidermidis, S. aureus* (MSSA and MRSA) and *P.aeruginosa,* of the strains identified above. The results showed Minimal Inhibitory concentration (MIC) and Minimal Eradication Concentration (MEC) for planktonic cultures. Testing of co-precipitate conducted after one hour reconstitution.

A summary of the in-use dilutions and concentrations required to inhibit and eradicate planktonic bacteria (MIC/MEC) for all the tested bacteria are provided in in Table 2:

**Table 2 : MIC/MEC for co-precipitate (antibiofilm complex) against four common biofilm forming pathogenic bacteria. Dilution expressed relative to in-use strength of 3.71g/l.**

| **Species Commencing bacterial count** | **MIC** | | **MEC** | |
|---|---|---|---|---|
| | **Dilution of neat** | **Concentration (mg/ml)** | **Dilution of neat** | **Concentration (mg/ml)** |
| *S*. *epidermidis* | | | | |
| 10⁷ | 1 in 20,000 | 0.0001855 | 1 in 5 | 0.742 |
| 10⁶ | 1 in 20,000 | 0.0001855 | | |
| 10⁵ | 1 in 20,000 | 0.0001855 | | |
| MSSA | | | | |
| 10⁷ | 1 in 10,000 | 0.000371 | 1 in 2.5 | 1.484 |
| 10⁶ | 1 in 25,000 | 0.0001484 | 1 in 5,000 | 0.000742 |
| 10⁵ | 1 in 25,000 | 0.0001484 | 1 in 5,000 | 0.000742 |
| MRSA | | | | |
| 10⁷ | 1 in 50,000 | 0.0000742 | 1 in 2.5 | 1.484 |
| 10⁶ | 1 in 50,000 | 0.0000742 | 1 in 2500 | 0.001484 |
| 10⁵ | 1 in 50,000 | 0.0000742 | 1 in 10,000 | 0.000371 |
| *P. aeruginosa* | | | | |
| 10⁷ | 1 in 50 | 0.0742 | 1 in 20 | 0.1855 |
| 10⁶ | 1 in 50 | 0.0742 | | |
| 10⁵ | 1 in 50 | 0.0742 | | |

The results shown in Table 2 were interpreted as demonstrating broad spectrum activity by the novel antibiofilm complex against the planktonic bacteria tested. Its inhibitory activity against gram positive organisms (most likely to be involved in DAI) is higher than when compared with *P. aeruginosa* (Gram negative). The dilution required for eradication, however, is considerably higher (e.g., a lower concentration) for *P. aeruginosa* as compared with the gram positives tested.

Experiment 1 also demonstrated Minimal Biofilm Inhibitory concentration (MBIC) and Minimal Biofilm Eradication Concentration (MBEC) for biofilm cultures during the testing of the novel antibiofilm complex conducted after one hour of reconstitution. A summary of the in-use dilutions and concentrations required to inhibit and eradicate biofilm bacteria (MBIC/MBEC) for all the tested bacteria are found in Table 3:

**Table 3: MBIC/MBEC for co-precipitate (antibiofilm complex) against 3 common biofilm forming pathogenic bacteria. Dilution expressed relative to in-use strength of 3.71g/l.**

| **Species Commencing bacterial count** | **MBIC** | | **MBEC** | |
|---|---|---|---|---|
| | **Dilution of neat** | **Concentration (mg/ml)** | **Dilution of neat** | **Concentration (mg/ml)** |
| *S*. *epidermidis* | | | | |
| 10⁷ CFU/well | 1 in 100 | 0.0371 | 1 in 5 | 0.742 |
| S. *aureus* MSSA | | | | |
| 10⁷ CFU/well | 1 in 20 | 0.1855 | 1 in 20 | 0.1855 |
| P. aeruginosa | | | | |
| 10⁷ CFU/well | 1 in 5 | 0.742 | 1 in 5 | 0.742 |

The results shown in Figure 3 were interpreted as demonstrating, in comparison to killing of planktonic bacteria, that the novel antibiofilm complex predictably required a higher concentration (and lesser dilution) to inhibit and kill biofilm bacteria. It is important to note the *in vitro* testing of biofilm bacteria was conducted in 100% tryptone soy broth, a high protein environment providing significant protein interference of antibiotic and antiseptic action.

### Experiment 2. The efficacy of co-precipitate (antibiofilm complex) when used against Staphylococcus aureus (MSSA) after 3 & 24 hours of reconstitution compared to reconstitution after 1 hour. (MIC, MEC, MBIC, MBEC)

Table 4 summarizes dilutions and concentrations required to inhibit *S*. *aureus* (Strain 25923) at 1 hour, 3 hours and 24 hours following reconstitution of co-precipitate.

**Table 4: The MIC/MEC/MBIC/MBEC for co-precipitate (antibiofilm complex) against S. aureus (strain 25923) at 1 hour versus 3 and 24 hours following reconstitution.**

| | **MIC Planktonic cells** | | **MEC Planktonic cells** | | **MBIC Biofilm cells** | | **MBEC Biofilm cells** | |
|---|---|---|---|---|---|---|---|---|
| | **Dilution** | **Conc. (mg/ml)** | **Dilution** | **Conc. (mg/ml)** | **Dilution** | **Conc. (mg/ml)** | **Dilution** | **Conc. (mg/ml)** |
| 1 hour | | | | | | | | |
| 10⁷ | 1 in 10,000 | 0.000371 | 1 in 2,000 | 0.001855 | | | | |
| 10⁶ | 1 in 10,000 | 0.000371 | 1 in 2,000 | 0.001855 | | | | |
| 10⁷ biofilm | | | | | 1 in 20 | 0.1855 | 1 in 20 | 0.1855 |
| 3 hours | | | | | | | | |
| 10⁷ | 1 in 10,000 | 0.000371 | 1 in 2,000 | 0.001855 | | | | |
| 10⁶ | 1 in 10,000 | 0.000371 | 1 in 2,000 | 0.001855 | | | | |
| 10⁷ biofilm | | | | | 1 in 20 | 0.1855 | 1 in 20 | 0.1855 |
| 24 hours | | | | | | | | |
| 10⁷ biofilm | | | | | 1 in 25 | 0.1484 | 1 in 25 | 0.1484 |

The results shown in Table 4 were interpreted as appearing to show no loss in efficacy in reconstitution of the novel antibiofilm complex at 1 hour versus 3 hours against the planktonic form of S. aureus. For S. aureus biofilm, the novel antibiofilm complex maintained its potency at both 3 hours and 24 hours following reconstitution, an added advantage in providing prolonged protection against biofilm for medical devices in situ. This also demonstrates stability in the novel antibiofilm complex following reconstitution at the 24 hour time point.

### Experiment 3. The efficacy of the physical mixture of PVP, iodine, gentamycin and rifampicin against S. aureus at 1 and 3 hour time point following reconstitution (MIC, MEC, MBIC, MBEC)

Table 5 summarizes dilutions and concentrations of reconstituted physical mixture of povidone iodine, rifampicin and gentamycin required to inhibit S. aureus (MIC/MEC/MBIC/MBEC) (Strain 25923) when added to cultures at 1 hour and 3 hours following reconstitution.

**Table 5 : MIC/MEC/MBIC/MBEC for physical mixture of povidone iodine, rifampicin and gentamycin against S. aureus (strain 25923) when tested at 1 hour and 3 hours following reconstitution.**

| | **MIC** | | **MEC** | | **MBIC** | | **MBEC** | |
|---|---|---|---|---|---|---|---|---|
| | Dilution | **Conc. (mg/ml)** | **Dilution** | **Conc. (mg/ml)** | **Dilution** | **Conc. (mg/ml)** | **Dilution** | **Conc. (mg/ml** |
| 1 hour | | | | | | | | |
| 10⁷ | 1 in 2000 | 0.001855 | 1 in 1000 | 0.00371 | | | | |
| 10⁷ biofilm | | | | | 1 in 20 | 0.1855 | 1 in 20 | 0.1855 |
| 3 hours | | | | | | | | |
| 10⁷ | 1 in 2000 | 0.001855 | 1 in 1000 | 0.00371 | | | | |
| 10⁷ biofilm | | | | | 1 in 20 | 0.1855 | 1 in 20 | 0.1855 |

The results of Table 5 were interpreted as follows. By comparison with the co-precipitate, the physical mixture of each of the parent ingredients showed significantly less potency against planktonic cultures of S. aureus. This indicates a synergy of action that is gained by chemically linking the antibiotics, iodine and povidone in the high-energy co-precipitate. This synergy may be the result of changes to solubility and membrane seeking properties of the antibiotics when bound into a co-precipitate with povidone. (See, Mirzabeigi MN, Sbitany H, Jandali S, Serletti JM. The Role of Postoperative Antibiotics in Reducing Biofilm-related Capsular Contracture in Augmentation Mammaplasty. Plastic and Reconstructive Surgery 2011; 128(1): 34e-5e.)

### Experiment 4. The efficacy of Betadine (povidone iodine) versus S. aureus and S. epidermidis immediately following dilution (MIC, MEC, MBIC, MBEC)

Testing was conducted using Betadine, containing povidone iodine 10% w/v equivalent to 1%w/v available iodine as the "in-use" neat solution. Table 6 summarizes dilutions and concentrations required to inhibit S. epidermidis planktonic cultures (MIC/MBE) and S. epidermidis and S. aureus biofilm cultures (MBIC/MBEC) (Strain 25923) for Betadine.

**Table 6: The efficacy of Betadine against Staphylococcus epidermidis and S. aureus as measured by the MIC/MEC/MBIC/MBEC.**

| | **MIC** | | **MEC** | | **MBIC** | | **MBEC** | |
|---|---|---|---|---|---|---|---|---|
| | **Dilution** | **Conc. (mg/ml)** | **Dilution** | **Conc. (mg/ml)** | **Dilution** | **Conc. (mg/ml)** | **Dilution** | **Conc. (mg/ml)** |
| *Staphylococcus epidermidis* | | | | | | | | |
| 10⁷ | 1 in 8 | 1.25 | 1 in 4 | 2.5 | | | | |
| 10⁷ biofilm | | | | | 1 in 8 | 1.25 | 1 in 4 | 2.5 |
| *Staphylococcus aureus* | | | | | | | | |
| 10⁷ biofilm | | | | | 1 in 10 | 1 | 1 in 5 | 2 |

The results of Table 6 were interpreted as demonstrating that a much higher concentration of Betadine was required to inhibit and kill planktonic *S*. *epidermidis* than the 1 in 20,000 dilution (Table 2) necessary for the novel antibiofilm complex. A slightly higher concentration of betadine was required to kill *S*. *epidermidis* biofilm compared with the novel antibiofilm complex (Table 3).

### Analysis of Experimental Results

While a 1 in 20 dilution of the novel antibiofilm complex killed *S*. *aureus*, Betadine required a 1 in 5 dilution. Furthermore, the concentration of free iodine in the novel antibiofilm complex is at 25% of the concentration of free iodine in betadine. Hence, one significant advantage of using the novel antibiofilm complex is that the total dose of free iodine required to achieve an equivalent kill is far less thereby reducing potential toxicity.

Collectively, from the data compiled in Tables 2 - 6, it is clear that the novel antibiofilm complex displays marked and broad activity against a variety of both planktonic and biofilm forming bacteria. The concentrations required to inhibit and eradicate the bacteria are far lower than currently used in clinical practice. This shows a significant chemical synergy produced from combining the three antibiotic moieties with the PVP backbone as a co-precipitate. The major advantage of using this as an anti-infective is the relatively lower dosage of antibiotics and iodine required to produce an equivalent kill as compared with each of these agents in isolation. This has the added advantage of protecting the host tissues for potential toxicity and reducing the development of resistance.

The activity of the novel antibiofilm complex appears to be stable at the 3 hour time point showing no immediate deterioration of antimicrobial effectiveness. For biofilm testing, activity at 24 hours still showed significant ability to both inhibit and kill biofilm bacteria in a protein rich environment. It would be interesting to test the product after a longer period (i.e., 7 days) following reconstitution to assess medium term efficacy. Its prolonged activity may be an added advantage to protect against the development of DAI once a prosthesis is in situ.

By comparison, the individual physical mixture of components whilst displaying some antibiotic properties showed less activity as compared with the co-precipitate. When compared with betadine (the current favored antiseptic skin preparation and pocket preparation for prevention of SSI/DAI), the co-precipitate showed equivalent activity at a much lower dose of free iodine. There are significant advantages to using the co-precipitate as a topical anti-infective as compared with betadine. Finally, the prolonged antibiofilm activity as displayed by MBIC and MBEC at 24 hours provides an added advantage to continue to protect a prosthetic in situ following surgical implantation. Further time point studies of co-precipitate efficacy should be performed.

Figure 2 provides a comparison of activity of synergistic co-precipitate (antibiofilm complex) vs betadine for MIC, MEC (planktonic 10⁷ bacteria S. aureus ATCC 25932 (MSSA)) and MBEC (biofilm 10⁵ bacteria S. aureus ATCC 25932 (MSSA)) showing significantly less dosage of co-precipitate required for both bacterial inhibition and eradication.

In sum, the experiments demonstrate that the chemical "smart bomb" embodied in the novel antibiofilm complex works. At a minimum, the following conclusions can be drawn from the experimentation:
1. The novel co-precipitate shows broad activity against S. epidermidis, S. aureus (MSSA and MRSA) and P. aeruginosa;
2. The co-precipitate is more active against planktonic bacteria but also is able to penetrate and eradicate biofilm;
3. The activity of the co-precipitate shows synergy with added potency when compared with individual components and when compared with povidone iodine in isolation;
4. The lower concentrations of antibiotic as a result may provide benefit in reducing host toxicity whilst being able to specifically target bacteria both in planktonic and sessile forms;
5. The activity of the co-precipitate is maintained at 24 hours following reconstitution indicating a long period of potency; and
6. There is potential to develop this compound into a powerful anti-infective with clinical use in both the prevention and treatment of SSI/DAI.

### Exemplary Clinical Applications

The novel antibiofilm complex embodying the present inventive principles is suitable for a wide range of uses, particularly in the reduction and control of SSI and DAI. The following examples demonstrate only a few of its potential uses, including (1) surgical site preparation; (2) surgical pocket preparation; (3) implant immersion prior to deployment; (4) treatment of established biofilm infection for implant salvage; and (5) as an antimicrobial coating on medical prosthetics.

A typical clinic application begins with the preparation of stock solution. For example, a pre-determined vial containing 3.78g of the inventive antibiofilm complex is opened under sterile conditions in the operating room. This antibiofilm complex is mixed with 20cc of sterile water for injection (WFI). The resultant solution is then added to 500cc of sterile WFI to provide stock solution, with a total volume of 520cc, for use in surgical pocket preparation and/or implant decontamination. Preferably, the stock solution is reconstituted as close to the time of use as possible.

During an exemplary surgical site preparation, the skin and mucosal surfaces are washed with the stock solution prior to incision. The solution is then left in contact for a minimum of 30 seconds following commencement of the surgical procedure.

A typical example of surgical pocket preparation is the deployment of a mammary prosthesis. Following dissection of a suitable submammary or submuscular (dual plane) pocket and prior to deployment of the mammary implant, 150cc of stock solution is drawn and injected into the pocket. The surgeon ensures that the solution is spread evenly over the entire pocket and the solution is left in situ for a minimum of 30 seconds prior to prosthesis deployment.

Another representative use is implant immersion prior to deployment. In this example, an implant (e.g., a mammary prosthesis) is removed under sterile conditions from packaging whilst the surgical pocket is being treated by stock solution. The prosthesis is immersed in 100cc of stock solution for a minimum of 30 seconds. The prosthesis is then removed from the solution and immediately deployed into the prepared surgical pocket. If the implant is to be excessively manipulated and/or removed from the cavity, consideration must be given to re-preparation of the pocket and re-immersion of the implant as described above.

A further example of the potential uses of the stock solution containing the inventive antibiofilm complex is the orthopaedic internal fixation for fracture. Following exposure and reduction of the fracture, 150cc of stock solution is delivered to the surgical site and surrounding skin access incision. The solution is left in contact with the tissues for a minimum of 30 seconds prior to the delivery and fixation of the orthopaedic plate/screws. During this time, any anticipated plate/screws should be placed into a sterile container with 150cc of stock solution and be in contact with the solution for at least 30 seconds prior to deployment.

### Summary

In sum, by appropriate formulation and lyophilization procedures, a novel solid novel antibiofilm complex (GAAB-1) offers the following advantages:
(1) It converts relatively insoluble antiseptics and antibiotics into water soluble membrane seeking agents possessing biofilm penetrating properties and excellent antimicrobial action;
(2) It exhibits a significant synergistic antimicrobial/antiseptic action which is greater than that of a simple equimolar mixture of its three pharmacologically active agents involved or their individual equimolar solutions. Its higher potency at lower concentrations than that of a mixture of its constituents results in lower toxicity of the product. The synergy may be the result of the improved solubility of its constituents, greater extent of deposition on biologic membrane due to the intimate association of the two antibodies with Povidone (a polymer which possesses membrane seeking properties that explain its cell cryoprotectant action);
(3) Possesses broad spectrum activity against common biofilm forming pathogenic bacteria even in the presence of a high protein environment which provides significant protein interference of antibiotic and antiseptic action;
(4) Provides fixed concentrations of antibiotic (antiseptic components) thereby standardizing the dosage delivery; and
(5) The activity of the product is maintained for 24 hours following reconstitution indicating a long period of potency.

## Claims

1. An antibiofilm comprising a complex of:
a polyvinylpyrrolidone (PVP) backbone;
iodine anchored to the PVP backbone;
a first antibiotic selected from the group of rifamyacins anchored to the PVP backbone; and
a second antibiotic selected from the group consisting of aminoglycosides anchored to the PVP backbone.

2. The antibiofilm of Claim 1, wherein the first antibiotic comprises Rifampin.

3. The antibiofilm of Claim 1, wherein the second antibiotic comprises Tobramycin or Gentamicin.

4. The antibiofilm of Claim 1, wherein the antibiofilm:
- is in the form of a powder; or
- is in the form of a solution derived from reconstitution from stable freeze dried powder.

5. A method of preparing an antibiofilm including a polyvinylpyrrolidone (PVP) backbone anchoring at least iodine and two antibiotics, comprising:
preparing a solution including Povidone-iodine, Rifampin, and an antibiotic selected from the group consisting of Tobramycin and Gentamicin; and
performing lyophilization to produce a lyophilized coprecipitate in powder form.

6. The method of Claim 5, wherein preparing a solution comprises, preparing a solution in 50:50 water including:
tertiary butyl alcohol (TBA);
about 2.5 grams of Rifampin;
about 80 milligrams of the antibiotic selected from the group consisting of Tobramycin and Gentamicin; and
about 1.2 grams of povidone-iodine (10%);
and wherein preparing the solution preferably comprises preparing a solution with about 5% to about 45% TBA.

7. The method of Claim 5, wherein performing lyophilization comprises:
introducing the solution into a chamber;
reducing the chamber temperature to about -25 °C;
holding the chamber temperature at about -25 °C for about 10 hours during a first freezing step;
reducing the chamber temperature to about -45 °C;
holding the chamber temperature at about -45 °C for about 5 hours during a second freezing step.

8. The method of Claim 5, wherein performing lyophilization comprises:
introducing the solution into a chamber;
during a freezing step, reducing the chamber temperature; and
evacuating the chamber to create a partial vacuum of about 13,3 Pa (100 mtorr).

9. The method of Claim 5, wherein performing lyophilization comprises:
introducing the solution into a chamber;
during a freezing step, reducing the chamber temperature;
evacuating the chamber to create a partial vacuum;
during a drying step, increasing the chamber temperature to about 20 °C; and
holding the chamber temperature at about 20 °C for about 30 hours;
and optionally further comprises:
during a second drying step, further increasing the chamber temperature to about 40 °C and increasing the pressure to about 20,0 Pa (150 mTorr); and
holding the chamber temperature and temperature at about 40 °C and about 20,0 Pa (150 mTorr) for about 12 hours.

10. The method of Claim 5, wherein performing lyophilization comprises:
introducing the solution into a chamber;
during a first freezing step:
reducing a chamber temperature to about -25 °C during a first ramping period; and
holding the chamber temperature at about -25 °C for about 10 hours;
during a second freezing step:
reducing the chamber temperature to about -45 °C during a second ramping period; and
holding the chamber temperature at about -45 °C for about 5 hours;
evacuating the chamber to create a chamber partial vacuum of about 13,3 Pa (100 mTorr);
during a first drying step:
increasing the chamber temperature during a third ramping period to about 20 °C; and
holding the chamber temperature at about 20 °C for about 30 hours; and
during a second drying step:
increasing the chamber temperature and pressure during a fourth ramping period to about 40 °C and 20,0 Pa (150 mTorr); and
holding the chamber temperature and pressure at about 20 °C and 20,0 Pa(150mTorr) for about 12 hours.

11. The method of Claim 10, wherein:
the first ramping period is about 1 hour;
the second ramping period is about 3 hours;
the third ramping period is about 3 hours; and
the fourth ramping period is about 1 hour.

## Patentansprüche

1. Antibiofilm umfassend einen Komplex aus:
einem Polyvinylpyrrolidon (PVP)-Rückgrat;
einem am PVP-Rückgrat verankerten Jod;
einem ersten Antibiotikum ausgewählt aus der Gruppe von am PVP-Rückgrat verankerten Rifamyacinen; und
einem zweiten Antibiotikum ausgewählt aus der Gruppe bestehend aus am PVP-Rückgrat verankerten Aminoglykosiden.

2. Antibiofilm nach Anspruch 1, wobei das erste Antibiotikum Rifampin umfasst.

3. Antibiofilm nach Anspruch 1, wobei das zweite Antibiotikum Tobramycin oder Gentamicin umfasst.

4. Antibiofilm nach Anspruch 1, wobei der Antibiofilm:
- in Form eines Pulvers vorliegt; oder
- in Form einer aus einer Rekonstitution aus stabilem gefriergetrocknetem Pulver abgeleiteten Lösung vorliegt.

5. Verfahren zur Herstellung eines Antibiofilms enthaltend ein Polyvinylpyrrolidon (PVP)-Rückgrat, welches zumindest Jod und zwei Antibiotika verankert, umfassend:
Herstellen einer Lösung enthaltend Povidon-Jod, Rifampin und ein Antibiotikum ausgewählt aus der Gruppe bestehend aus Tobramycin und Gentamicin; und
Durchführen einer Lyophilisierung, um ein lyophilisiertes Copräzipitat in Pulverform zu erzeugen.

6. Verfahren nach Anspruch 5, wobei das Herstellen einer Lösung das Herstellen einer Lösung in 50:50 Wasser umfasst, enthaltend:
Tertiärbutylalkohol (TBA);
etwa 2,5 Gramm von Rifampin;
etwa 80 Milligramm des Antibiotikums ausgewählt aus der Gruppe bestehend aus Tobramycin und Gentamicin; und
etwa 1,2 Gramm von Povidon-Jod (10%);
und wobei das Herstellen der Lösung vorzugsweise das Herstellen einer Lösung mit etwa 5 % bis etwa 45 % TBA umfasst.

7. Verfahren nach Anspruch 5, wobei das Durchführen einer Lyophilisierung umfasst:
Einführen der Lösung in eine Kammer;
Verringern der Kammertemperatur auf etwa -25 °C;
Halten der Kammertemperatur bei etwa -25 °C für etwa 10 Stunden während eines ersten Gefrierschritts;
Verringern der Kammertemperatur auf etwa -45 °C;
Halten der Kammertemperatur bei etwa -45 °C für etwa 5 Stunden während eines zweiten Gefrierschritts.

8. Verfahren nach Anspruch 5, wobei das Durchführen einer Lyophilisierung umfasst:
Einführen der Lösung in eine Kammer;
Verringern der Kammertemperatur während eines Gefrierschritts; und
Evakuieren der Kammer, um ein Teilvakuum von etwa 13,3 Pa (100 mTorr) zu erzeugen.

9. Verfahren nach Anspruch 5, wobei das Durchführen einer Lyophilisierung umfasst:
Einführen der Lösung in eine Kammer;
Verringern der Kammertemperatur während eines Gefrierschritts;
Evakuieren der Kammer zur Erzeugung eines Teilvakuums;
während eines Trocknungsschritts Erhöhen der Kammertemperatur auf etwa 20 °C; und Halten der Kammertemperatur bei etwa 20 °C für etwa 30 Stunden;
und optional ferner umfasst:
während eines zweiten Trocknungsschritts weiteres Erhöhen der Kammertemperatur auf etwa 40 °C und Erhöhen des Drucks auf etwa 20,0 Pa (150 mTorr); und
Halten der Kammertemperatur und Temperatur bei etwa 40 °C und etwa 20,0 Pa (150 mTorr) für etwa 12 Stunden.

10. Verfahren nach Anspruch 5, wobei das Durchführen einer Lyophilisierung umfasst:
Einführen der Lösung in eine Kammer;
während eines ersten Gefrierschritts:
Verringern einer Kammertemperatur auf etwa -25 °C während einer ersten Rampenzeit; und
Halten der Kammertemperatur bei etwa -25 °C für etwa 10 Stunden;
während eines zweiten Gefrierschritts:
Verringern der Kammertemperatur auf etwa -45 °C während einer zweiten Rampenzeit; und
Halten der Kammertemperatur bei etwa -45 °C für etwa 5 Stunden;
Evakuieren der Kammer, um ein Teilvakuum von etwa 13,3 Pa (100 mTorr) in einer Kammer zu erzeugen;
während eines ersten Trocknungsschritts:
Erhöhen der Kammertemperatur während einer dritten Rampenzeit auf etwa 20 °C; und
Halten der Kammertemperatur bei etwa 20 °C für etwa 30 Stunden; und
während eines zweiten Trocknungsschritts:
Erhöhen der Kammertemperatur und des Drucks während einer vierten Rampenzeit auf etwa 40 °C und 20,0 Pa (150 mTorr); und
Halten der Kammertemperatur und des Drucks bei etwa 20 °C und 20,0 Pa (150 mTorr) für etwa 12 Stunden.

11. Verfahren nach Anspruch 10, wobei:
die erste Rampenzeit etwa eine Stunde beträgt;
die zweite Rampenzeit etwa drei Stunden beträgt;
die dritte Rampenzeit etwa drei Stunden beträgt; und
die vierte Rampenzeit etwa eine Stunde beträgt.

## Revendications

1. Antibiofilm comprenant un complexe consistant en:
un squelette de polyvinylpyrrolidone (PVP);
de l'iodine ancrée au squelette de PVP;
un premier antibiotique choisi dans le groupe des rifamyacins ancrés dans le squelette de PVP;
et
un deuxième antibiotique choisi dans le groupe constitué par les aminoglycosides ancrés dans le squelette de PVP.

2. Antibiofilm selon la revendication 1, dans lequel le premier antibiotique comprend de la Rifampine

3. Antibiofilm selon la revendication 1, dans lequel le deuxième antibiotique comprend la Tobramycine ou la Gentamicine.

4. Antibiofilm selon la revendication 1, dans lequel l'antibiofilm:
- est sous forme d'une poudre; ou
- est sous la forme d'une solution dérivée de la reconstitution à partir d'une poudre lyophilisée stable.

5. Procédé de préparation d'un antibiofilm comprenant un squelette de polyvinylpyrrolidone (PVP) ancrant au moins l'iodine et deux antibiotiques, comprenant:
la préparation d'une solution incluant Povidone-iodine, Rifampine et un antibiotique choisi dans le groupe consistant en Tobramycine et Gentamicine; et
la réalisation d'une lyophilisation pour produire un coprécipité lyophilisé sous forme de poudre.

6. Procédé selon la revendication 5, dans lequel la préparation d'une solution comprend, la préparation d'une solution dans 50:50 d'eau comprenant:
l'alcool butylique tertiaire (TBA);
environ 2,5 grammes de Rifampine;
environ 80 milligrammes de l'antibiotique choisi dans le groupe consistant en Tobramycine et Gentamicine; et
environ 1,2 gramme de povidone-iodine (10%);
et dans lequel la préparation de la solution comprend de préférence la préparation d'une solution avec environ 5% à environ 45% de TBA.

7. Procédé selon la revendication 5, dans lequel la réalisation d'une lyophilisation comprend:
l'introduction de la solution dans une chambre;
la réduction de la température de la chambre à environ -25 °C;
le maintien de la température de la chambre à environ -25 °C pendant environ 10 heures pendant une première étape de congélation;
la réduction de la température de la chambre à environ -45 °C;
le maintien de la température de la chambre à environ -45 °C pendant environ 5 heures pendant une deuxième étape de congélation.

8. Procédé selon la revendication 5, dans lequel la réalisation d'une lyophilisation comprend:
l'introduction de la solution dans une chambre;
pendant une période de congélation, la réduction de la température de la chambre; et
l'évacuation de la chambre pour créer un vide partiel d'environ 13,3 Pa (100 mTorr).

9. Procédé selon la revendication 5, dans lequel la réalisation d'une lyophilisation comprend:
l'introduction de la solution dans une chambre;
pendant une période de congélation, la réduction de la température de la chambre;
l'évacuation de la chambre pour créer un vide partiel;
pendant une étape de séchage, l'augmentation de la température de la chambre à environ 20 °C; et le maintien de la température de la chambre à environ 20 °C pendant environ 30 heures.
et éventuellement en outre comprend:
pendant une deuxième étape de séchage, l'augmentation encore la température de la chambre à environ 40 °C et l'augmentation de la pression à environ 20,0 Pa (150 mTorr); et
le maintien de la température de la chambre et la température à environ 40 °C et environ 20,0 Pa (150 mTorr) pendant environ 12 heures.

10. Procédé selon la revendication 5, dans lequel la réalisation d'une lyophilisation comprend:
l'introduction de la solution dans une chambre;
pendant une première étape de séchage:
la réduction de la température de la chambre à environ -25 °C lors d'une première période de rampe; et
le maintien de la température de la chambre à environ -25 °C pendant environ 10 heures;
pendant une deuxième étape de congélation:
la réduction de la température de la chambre à environ -45 °C lors d'une deuxième période de rampe; et
le maintien de la température de la chambre à environ -45 °C pendant environ 5 heures;
l'évacuation de la chambre pour créer un vide partiel d'environ 13,3 Pa (100 mTorr) ;
pendant une première étape de séchage:
l'augmentation de la température de la chambre pendant une troisième période de rampe à environ 20 °C; et
le maintien de la température de la chambre à environ 20 °C pendant environ 30 heures; et
pendant une deuxième étape de séchage:
l'augmentation de la température de la chambre et de la pression pendant une quatrième période de rampe à environ 40 °C et 20,0 Pa (150 mTorr); et
le maintien de la température de la chambre et de la pression à environ 20 °C et 20,0 Pa (150 mTorr) pendant environ 12 heures.

11. Procédé selon la revendication 10, dans lequel:
la première période de rampe est environ 1 heure;
la deuxième période de rampe est environ 3 heures;
la troisième période de rampe est environ 3 heures; et
la quatrième période de rampe est environ 1 heure.
